# EUROPEAN PATENT APPLICATION

(11) **EP 3 760 100 A1**
(43) Date of publication of application: **06.01.2021**
(21) Application number: 20183357.1
(22) Date of filing: 01.07.2020
(51) Int. Cl.: A61B 1/303, A61B 1/05, A61B 1/005, A61B 1/00, A61B 1/008

(54) **COLPOSCOPE FOR SCREENING FOR CERVICAL CANCER**

(30) Priority: 02.07.2019 US 201962869694 P; 20.05.2020 US 202016879230
(71) Applicant: Covidien LP, Mansfield, MA 02048 (US)
(72) Inventor: KUMAR, Arun, TN 600091 Chennai (IN)
(74) Representative: Maschio, Antonio

(57) **Abstract**

A colposcope includes a handle assembly, an elongate member extending distally from the handle assembly, and a camera assembly including a neck portion coupled to the elongate member, and a body portion. The handle assembly includes a grip, a trigger movable relative to the grip, and an articulation dial operatively coupled with the body portion of the camera assembly. The elongate member includes a plurality of lumens. The body portion of the camera assembly includes an image sensor assembly having an image sensor and light emitters. The image sensor assembly is operatively coupled with the trigger of the handle assembly such that when the trigger is actuated the image sensor captures images of a target.

## Description

### CROSS-REFERENCE TO RELATED APPLICATION

This application claims the benefit of and priority to U.S. Provisional Patent Application Serial No. 62/869,694 filed July 2, 2019, the entire disclosure of which is incorporated by reference herein.

### FIELD

The disclosure relates to a surgical device and, more particularly, to a portable colposcope for use in screening for cervical cancer.

### BACKGROUND

The screening for cervical cancer is conducted through an in-vivo colposcope that captures the images of the cervix with the help of a vaginal speculum to dilate the end of the cervical space. Other tests include papanikolaou smear (Pap), in which, a sample of the cervical epithelial cells are taken and tested for abnormal morphology to determine the presence of carcinomic growth. The former approach has associated patient discomfort and/or pain during the screening and is highly adopted in urban hospital care settings. The latter approach is a simpler one, but has a lead time associated with the diagnosis.

An effective cancer screening and diagnostic program often requires both sophisticated and expensive medical facilities with well-trained and experienced medical staff. Affordability to the above diagnostic methods is centered highly in urban population and access to these methods is limited in the rural settings and in developing countries, as there is often an absence of appropriate medical infrastructure and resources to support the organized screening and diagnostic programs.

### SUMMARY

In accordance with an embodiment of the disclosure, a colposcope includes a handle assembly, an elongate member extending distally from the handle assembly, and a camera assembly including a neck portion coupled to the elongate member, and a body portion. The handle assembly includes a grip, a trigger movable relative to the grip, and an articulation dial operatively coupled with the body portion of the camera assembly. The elongate member includes a plurality of lumens. The body portion of the camera assembly includes an image sensor assembly having an image sensor and light emitters. The image sensor assembly is operatively coupled with the trigger of the handle assembly such that when the trigger is actuated the image sensor captures images of a target.

In an embodiment, the camera assembly may further include a fluid outlet for supplying fluid to a target tissue.

In another embodiment, the handle assembly may further include a fluid chamber configured to store fluid therein. A first lumen of the plurality of lumens may be configured to provide fluid communication between the fluid chamber and the fluid outlet of the camera assembly.

In yet another embodiment, the handle assembly may further include a fluid loading port configured to receive fluid therethrough. The fluid loading port may be in communication with the fluid chamber.

In still yet another embodiment, the handle assembly may further include an infusion button to supply the fluid in the fluid chamber to the target tissue through the fluid outlet of the camera assembly.

In an embodiment, the colposcope may further include a pair of articulation cables interconnecting the articulation dial and the body portion of the camera assembly such that axial displacement of at least one of the articulation cables causes articulation of the camera assembly.

In another embodiment, the pair of articulation cables may extend through a second lumen of the plurality of lumens.

In yet another embodiment, the light emitters may surround the image sensor.

In still yet another embodiment, the image sensor assembly may further include color filters.

In still yet another embodiment, the image sensor assembly may further include a pair of convex lenses to focus an object image of tissue on the image sensor.

In an embodiment, the colposcope may further include cables interconnecting the camera assembly and the handle assembly to transfer power and video data therebetween. The cables may extend through a third lumen of the plurality of lumens.

In another embodiment, the camera assembly may include a hemispherical dome-shaped acrylic housing.

In yet another embodiment, the handle assembly may further include a motor operatively coupled with the articulation dial such that actuation of the motor through rotation of the articulation dial causes axial displacement of at least one of the articulation cables.

In still yet another embodiment, the elongate member may have a tubular structure.

In still yet another embodiment, the elongate member may be formed of silicone.

In still yet another embodiment, the articulation dial may be rotatable about a longitudinal axis defined by the elongate member.

In an embodiment, the handle assembly may further include a battery pack detachably received in the grip. The battery pack may be configured to supply power to the camera assembly.

In another embodiment, the image sensor of the camera assembly may be a charge-coupled device.

In accordance with another embodiment of the disclosure, a kit for performing colposcopy includes a colposcope and an electronic device that is connectable to the colposcope. The colposcope includes a handle assembly, an elongate member extending distally from the handle assembly, and a camera assembly including a neck portion coupled to the elongate member, and a body portion. The handle assembly includes a grip, a trigger movable towards and away from the grip, and an articulation dial operatively coupled with the body portion of the camera assembly. The elongate member includes lumens. The body portion of the camera assembly includes an image sensor assembly having an image sensor and light emitters. The image sensor assembly is operatively coupled with the trigger of the handle assembly such that when the trigger is actuated, the image sensor captures images of a target tissue. The electronic device is configured to display the target tissue.

In an embodiment, the electronic device may be wirelessly connectable to the colposcope.

In another embodiment, the colposcope may further include a memory to store images of the target tissue captured by the image sensor.

In still yet another embodiment, the camera assembly may be movable between a first position, in which, the camera assembly is aligned with the longitudinal axis of the elongate member, and a second position in which at least a portion of the camera assembly is offset from the longitudinal axis of the elongate member.

### BRIEF DESCRIPTION OF THE DRAWINGS

Various embodiments of the disclosure are described hereinbelow with reference to the drawings, wherein:
FIG. 1 is a perspective view of a colposcope in accordance with an embodiment of the disclosure;
FIG. 1A is a partial perspective view of the colposcope, illustrating a camera assembly in an articulated position;
FIG. 2 is a cross-sectional view of the colposcope of FIG. 1, illustrating use with an electronic device;
FIG. 3 is a partial side cross-sectional view of a camera assembly of FIG. 2; and
FIG. 4 is a block diagram of digital image acquisition electronics of the colposcope of FIG. 1.

### DETAILED DESCRIPTION

Embodiments of the disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the disclosure in unnecessary detail.

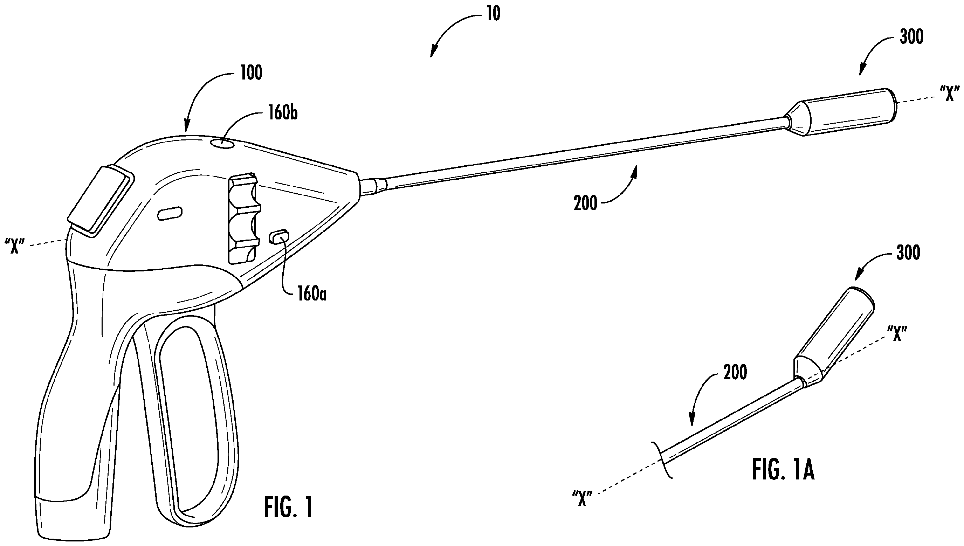

FIG. 1 illustrates a colposcope 10 in accordance with an embodiment of the disclosure. The colposcope 10 is a portable device that screens for cervical cancer through imaging of the cervix and the endocervical space. Visualization of the cervix to discern the abnormal cell growth provides faster identification of the phase and grade of the neoplasmic growth, which, in turn, allows for expedited treatment. In addition, the colposcope 10 may reduce patient discomfort or pain during screening by eliminating the need for a vaginal speculum. Furthermore, the low cost of the colposcope 10 may improve screening in areas that lack appropriate medical infrastructure and resources to support the organized screening and diagnostic programs.

The colposcope 10 includes a handle assembly 100, an elongate member 200 extending distally from the handle assembly 100, and a camera assembly 300 detachably coupled to the elongate member 200. The colposcope 10 may be configured as a single-use device that is discarded after use or sent to a manufacturer for reprocessing, a reusable device capable of being cleaned and/or sterilized for repeated use by the end-user, or a partially-single-use, partially-reusable device. With respect to partially-single-use, partially-reusable configurations, the handle assembly 100 and the elongate member 200 may be configured as a cleanable/sterilizable, reusable component, while the camera assembly 300 is configured as a single-use, disposable/reprocessable component, or vice versa. In either of the above configurations, the handle assembly 100, the elongate member 200, and the camera assembly 300 are configured to releasably engage each other to facilitate disposal/reprocessing of any single-use components and cleaning and/or sterilization of any reusable component. Furthermore, enabling releasable engagement of the components allows for use of different handle assemblies and/or camera assemblies with the elongate member 200.

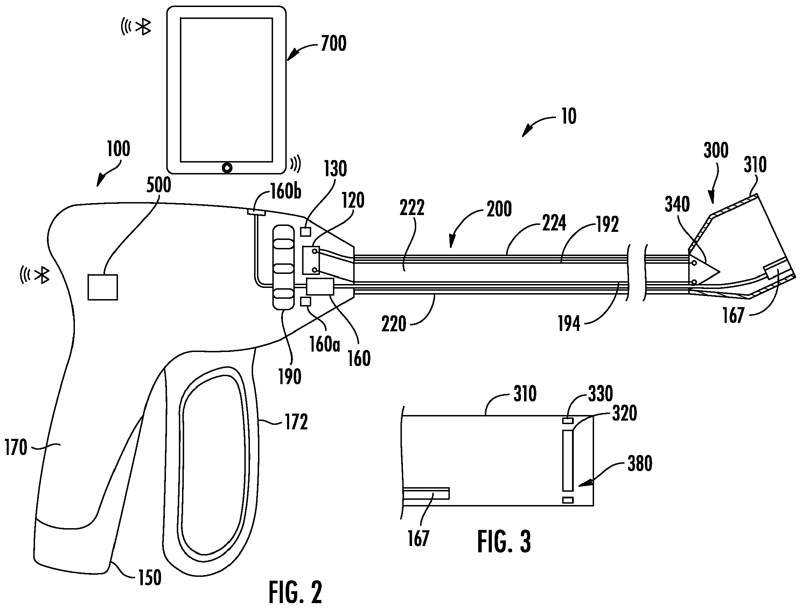

With reference to FIG. 2, the handle assembly 100 includes a grip 170 and a trigger 172 operatively associated with an image sensor 320 (FIG. 3) of the camera assembly 300 such that when the trigger 172 is squeezed towards the grip 170 by the clinician, the trigger 172 actuates the image sensor 320 to capture images and/or videos of a target tissue. The handle assembly 100 includes a battery pack 150 detachably received in the grip 170 to supply power to the colposcope 10.

The handle assembly 100 further includes an articulation dial 190 operatively coupled to the camera assembly 300 via, e.g., a pair of cables 192, 194. Rotation of the articulation dial 190 about a longitudinal axis "X-X" (FIG. 1) defined by the elongate member 200 articulates the camera assembly 300 between a neutral position, in which, the camera assembly 300 is aligned with the longitudinal axis "X-X" and an articulated position, in which, at least a portion of the camera assembly 300 is offset from the longitudinal axis "X-X". The articulation dial 190 may be operatively coupled with, e.g., a motor 120, to facilitate axial displacement of the cables 192, 194 when the articulation dial 190 is rotated in order to articulate the camera assembly 300.

The handle assembly 100 further includes a fluid chamber 160 configured to store fluid such as, e.g., saline or acetic acid, therein, and an infusion button 160a to supply the fluid in the fluid chamber 160 to a target tissue through a fluid outlet 167 on the camera assembly 300. The handle assembly 100 may further include a fluid loading port 160b in communication with the fluid chamber 160 to supply fluid to the fluid chamber 160.

FIG. 2 illustrates the colposcope 10 configured to be operatively coupled to an electronic device 700. In particular, the colposcope 10 and the electronic device 700 may be part of a kit provided for use by the clinician. For example, the electronic device 700 may display the images/videos captured by the image sensor 320 (FIG. 3) and/or real-time images/videos of the target tissue. The electronic device 700 may include any type of conventional computer, for example, a desktop computer or a laptop computer. A typical electronic device is a wireless data access-enabled device (e.g., an iPHONE® smart phone, a BLACKBERRY® smart phone, a NEXUS ONE™ smart phone, an iPAD® device, or the like) that is capable of sending and receiving data in a wireless manner using protocols like the Internet Protocol, or IP, and the wireless application protocol, or WAP. Wireless data access is supported by many wireless networks, including, but not limited to, CDPD, CDMA, GSM, PDC, PHS, TDMA, FLEX, ReFLEX, iDEN, TETRA, DECT, DataTAC, Mobitex, EDGE and other 2G, 3G, 4G and LTE technologies, and it operates with many handheld device operating systems, such as PalmOS, EPOC, Windows CE, FLEXOS, OS/9, JavaOS, iOS and Android. Typically, these devices use graphical displays and can access the Internet (or other communications network) on so-called mini- or micro-browsers, which are web browsers with small file sizes that can accommodate the reduced memory constraints of wireless networks. In a representative embodiment, the electronic device 700 is a cellular telephone or smart phone that operates over GPRS (General Packet Radio Services), which is a data technology for GSM networks. In addition to a conventional voice communication, a given electronic device 700 can communicate with another such device via many different types of message transfer techniques, including SMS (short message service), enhanced SMS (EMS), multi-media message (MMS), email WAP, paging, or other known or later-developed wireless data formats.

The handle assembly 100 further includes digital image acquisition electronics 500 that supports dual functionality of transferring processed image data from the image sensor 320 (FIG. 3) of the camera assembly 300 to the electronic device 700 and also to power the image sensor assembly 380 (FIG. 3). The handle assembly 100 may include, e.g., a flash memory, to store the captured images and/or videos until communicated to the electronic device 700 via wireless interface such as, e.g., Bluetooth™ or WiFi, or via the universal serial bus (USB) standard. In an embodiment, a network interface is configured to couple the colposcope 10 and the electronic device 700 to a network, such as a wired network, a wireless network, a local area network (LAN), a wide area network (WAN), a wireless mobile network, a Bluetooth™ network, the Internet, and/or other types of network. Captured images and/or videos may be suitably stored and processed. In an example, the images may be communicated or downloaded to a server for remote expert diagnosis. For example, the colposcope 10 and an electronic device 700 may implement a multimodal imaging technique to leverage intrinsic contrast from changes in collagen content through auto-fluorescent imaging and narrow band imaging of the neo-vascularization associated with progressively worsening cervical lesions derived from spectroscopic and ratiometric methods. It is contemplated that the electronic device 700 may be configured to control the operation of the colposcope 10, to process captured images and/or videos, and to interface with a user, such as a clinician.

The electronic device 700 may be communicatively connected to a remote server for communication of data and captured images for processing. In a representative embodiment, an electronic device, such as an e-book reader, is connectable (for example, via WAP) to a transmission functionality that varies depending on implementation. Embodiments in accordance with the present disclosure may be implemented in other and next-generation mobile networks and devices as well. The electronic device 700 is the physical equipment used by the end user, typically a subscriber to the wireless network. Typically, a mobile device is a 2.5G-compliant device, 3G-compliant device, or 4G-compliant device that includes a subscriber identity module (SIM), which is a smart card that carries subscriber- specific information, mobile equipment (e.g., radio and associated signal processing devices), a user interface (or a man-machine interface (MMI)), and one or more interfaces to external devices (e.g., computers, PDAs, and the like).

The elongate member 200 extends from the handle assembly 100. The elongate member 200 may include an annular tube. In particular, the elongate member 200 may be formed of a flexible material such as, e.g., silicone, to accommodate the anatomical structure of the endocervical space during insertion of the elongate member 200 into the endocervical space. The elongate member 200 includes first, second and third lumens 220, 222, 224. The first lumen 220 provides a fluid pathway between the fluid chamber 160 of the handle assembly 100 and the fluid outlet 167 on the camera assembly 300. The second lumen 222 is configured to receive articulation cables 192, 194 therethrough. The second lumen 222 is configured to enable axial displacement of the articulation cables 192, 194. The third lumen 224 is configured to receive cables to transfer power and video data between the handle assembly 100 and the camera assembly 300.

The camera assembly 300 includes a body portion 310 having, e.g., a cylindrical profile, and a neck portion 340. In an embodiment, the body portion 310 may include a hemispherical dome-shaped acrylic housing to enable circular field of view. The neck portion 340 is detachably coupled to the elongate member 200, and the body portion 310 is, e.g., pivotably, coupled to the neck portion 340. The articulation cables 192, 194 are operatively secured to the body portion 310 such that axial displacement of at least one of the articulation cables 192, 194 causes articulation of the body portion 310. Under such a configuration, rotation of the articulation dial 190 (FIG. 2) effects articulation of the image sensor assembly 380 of the body portion 310 relative to the longitudinal axis "X-X" (FIG. 1). Reference may be made to U.S. Patent No. 8,628,545, the entire contents of which is incorporated herein by reference, for a detailed discussion of the construction and operation of an exemplary articulation mechanism for articulating the camera assembly 300. The body portion 310 includes an image sensor assembly 380 including an image sensor 320 and light emitters 330. The image sensor 320 may be a charge-coupled device (CCD) based image sensor to capture the image data of the illuminated cervical space. The image sensor 320 may capture and store high-resolution, multimodal images of the cervix for post-hoc analysis by a clinician in a remote location. The image sensor 320 may be centered on a printed circuit assembly and the light emitters 330 may be arranged about the image sensor 310 to get a circular field of view. The image sensor assembly 310 may house series of color filters and pair of convex lenses to focus an object image of tissue. In addition, a refractive image condenser may enclose the image sensor 320 and the light emitters 330 to concentrate the optical information and get a better field view. The light emitters 330 may be high power light-emitting diodes (LEDs) to illuminate the cervix. Preferably, the light emitters emit a light that is white in color.

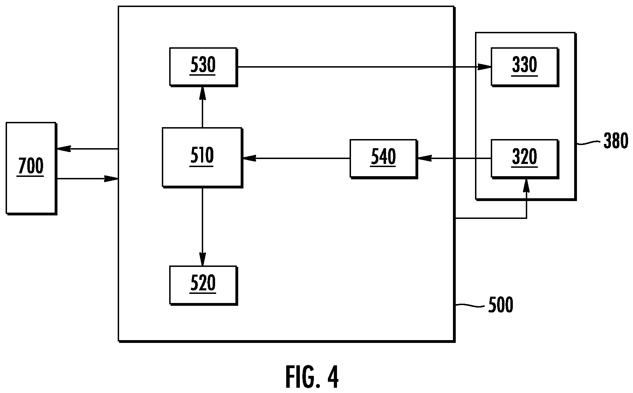

With reference to FIG. 4, the digital image acquisition electronics 500 of the handle assembly 100 includes a digital video processing block 510, an articulation controller 520, and an illumination controller 530. The digital video processing block 510 of the handle assembly 100 communicates with the image sensor assembly 380 of the camera assembly 300 for supplying power and receiving digital video frame data via serial interface packets. The digital video processing block 510 is realized through a de-serializer 540 and a video transfer controller of the processing block 510. The serial video data packets are de-serialized and fed into the video transfer controller for parsing the data to be sent to the mobile device 700 via a wireless interface such as, e.g., Bluetooth™ or through a USB interface. The illumination controller 530 drives the light emitters 330 that provide the required illumination to view the endocervical space. Further, the electronic device 700 may suitably power the light emitters 330 and the image sensor 320 via a cable.

In use, the clinician places the colposcope 10 in the endocervical space. At this time, the light emitters 330 may be used to illuminate the endocervical space. The endocervical space, the cervix, or a target tissue may be observed in real-time through the electronic device 700, e.g., wirelessly, connected to the colposcope 10. Alternatively, the colposcope 10 may be used as a stand-alone device without being connected to the electronic device 700. In an embodiment, the images and/or videos may be stored in the memory of the colposcope 10 and may be downloaded to the electronic device 700 when connected. The clinician may operate the articulation dial 190 to articulate the camera assembly 300 to place the image sensor 320 in a more desirable position. At this time, the clinician may apply, e.g., acetic acid, to the target tissue in order to induce contrast in, e.g., columnar epithelial cells of the cervical space which is suspected for malignancy. The clinician may actuate the trigger 172 to capture images and/or videos of the target tissue.

The colposcope 10 provides high resolution images and/or videos that enable an effective, low cost way to screen for cervical cancer without using a colposcopic speculum, thereby reducing discomfort and/or pain to a patient. The images and/or video captured by the colposcope 10 may be assessed by a clinician for any conditions of, e.g., dysplasia and neoplasmic growth, in suspicious cases, on the electronic device 700 by the on-site clinician or by a clinician in a remote location. It is contemplated that the components of the colposcope 10 may utilize mathematical algorithms to aid in a probabilistic heat map for highly suspicious lesion locations. In this manner, the colposcope 10 may further enhance reliable screening of cervical cancer in low-resource settings without causing great pain and/or discomfort to the patient.

Although the illustrative embodiments of the disclosure have been described herein with reference to the accompanying drawings, the above description, disclosure, and figures should not be construed as limiting, but merely as exemplifications of particular embodiments. It is further contemplated that the colposcope 10 may be adapted for use in robotic surgery.

It is also to be appreciated that the disclosure may be utilized in a number of applications including ligating tissue, hernia mesh repair, and in conjunction with implant drug delivery systems or procedures involving positioning of surgical or implantable devices in patients. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A colposcope comprising:
   a handle assembly including:
      a grip;
      a trigger movable relative to the grip; and
      an articulation dial;
   an elongate member extending distally from the handle assembly, the elongate member including a plurality of lumens; and
   a camera assembly including a neck portion coupled to the elongate member, and a body portion operatively coupled with the articulation dial, the body portion including an image sensor assembly having an image sensor and light emitters, the image sensor assembly operatively coupled with the trigger such that when the trigger is actuated the image sensor captures images of a target.
2. The colposcope according to paragraph 1, wherein the camera assembly further includes a fluid outlet for supplying fluid to a target tissue.
3. The colposcope according to paragraph 2, wherein the handle assembly further includes a fluid chamber configured to store fluid therein, a first lumen of the plurality of lumens configured to provide fluid communication between the fluid chamber and the fluid outlet of the camera assembly.
4. The colposcope according to paragraph 1, wherein the handle assembly further includes a fluid loading port configured to receive fluid therethrough, the fluid loading port in communication with the fluid chamber.
5. The colposcope according to paragraph 2, wherein the handle assembly further includes an infusion button to supply the fluid in the fluid chamber to the target tissue through the fluid outlet of the camera assembly.
6. The colposcope according to paragraph 1, further comprising a pair of articulation cables interconnecting the articulation dial and the body portion of the camera assembly such that axial displacement of at least one of the articulation cables causes articulation of the camera assembly.
7. The colposcope according to paragraph 6, wherein the pair of articulation cables extends through a second lumen of the plurality of lumens.
8. The colposcope according to paragraph 1, wherein the light emitters surround the image sensor.
9. The colposcope according to paragraph 1, wherein the image sensor assembly further includes color filters.
10. The colposcope according to paragraph 1, wherein the image sensor assembly further includes a pair of convex lenses to focus an object image of tissue on the image sensor.
11. The colposcope according to paragraph 1, further comprising cables interconnecting the camera assembly and the handle assembly to transfer power and video data therebetween, the cables extending through a third lumen of the plurality of lumens.
12. The colposcope according to paragraph 1, wherein the camera assembly includes a hemispherical dome-shaped acrylic housing.
13. The colposcope according to paragraph 1, wherein the handle assembly further includes a motor operatively coupled with the articulation dial such that actuation of the motor through rotation of the articulation dial causes axial displacement of at least one of the articulation cables.
14. The colposcope according to paragraph 1, wherein the elongate member has a tubular structure.
15. The colposcope according to paragraph 1, wherein the elongate member is formed of silicone.
16. The colposcope according to paragraph 1, wherein the articulation dial is rotatable about a longitudinal axis defined by the elongate member.
17. The colposcope according to paragraph 1, wherein the handle assembly further includes a battery pack detachably received in the grip, the battery pack configured to supply power to the camera assembly.
18. The colposcope according to paragraph 1, wherein the image sensor of the camera assembly is a charge-coupled device.
19. A kit for performing colposcopy comprising:
   a colposcope comprising:
      a handle assembly including:
         a grip;
         a trigger movable towards and away from the grip; and
         an articulation dial;
      an elongate member extending distally from the handle assembly, the elongate member including lumens; and
      a camera assembly including a neck portion coupled to the elongate member, and a body portion operatively coupled with the articulation dial, the body portion including an image sensor assembly having an image sensor and light emitters, the image sensor assembly operatively coupled with the trigger such that when the trigger is actuated the image sensor captures images of a target tissue; and
   an electronic device connectable to the colposcope, wherein the electronic device is configured to display the target tissue.
20. The kit according to paragraph 19, wherein the electronic device is wirelessly connectable to the colposcope.
21. The kit according to paragraph 19, wherein the colposcope further includes a memory to store images of the target tissue captured by the image sensor.
22. The kit according to paragraph 19, wherein the camera assembly is movable between a first position, in which, the camera assembly is aligned with the longitudinal axis of the elongate member, and a second position in which at least a portion of the camera assembly is offset from the longitudinal axis of the elongate member.

## Claims

1. A colposcope comprising:
a handle assembly including:
a grip;
a trigger movable relative to the grip; and
an articulation dial;
an elongate member extending distally from the handle assembly, the elongate member including a plurality of lumens; and
a camera assembly including a neck portion coupled to the elongate member, and a body portion operatively coupled with the articulation dial, the body portion including an image sensor assembly having an image sensor and light emitters, the image sensor assembly operatively coupled with the trigger such that when the trigger is actuated the image sensor captures images of a target.

2. The colposcope according to claim 1, wherein the camera assembly further includes a fluid outlet for supplying fluid to a target tissue; preferably wherein the handle assembly further includes a fluid chamber configured to store fluid therein, a first lumen of the plurality of lumens configured to provide fluid communication between the fluid chamber and the fluid outlet of the camera assembly.

3. The colposcope according to claim 1 or claim 2, wherein the handle assembly further includes a fluid loading port configured to receive fluid therethrough, the fluid loading port in communication with the fluid chamber.

4. The colposcope according to claim 2, wherein the handle assembly further includes an infusion button to supply the fluid in the fluid chamber to the target tissue through the fluid outlet of the camera assembly.

5. The colposcope according to any preceding claim, further comprising a pair of articulation cables interconnecting the articulation dial and the body portion of the camera assembly such that axial displacement of at least one of the articulation cables causes articulation of the camera assembly; preferably wherein the pair of articulation cables extends through a second lumen of the plurality of lumens.

6. The colposcope according to any preceding claim, wherein the light emitters surround the image sensor; and/or wherein the image sensor assembly further includes color filters; and/or wherein the image sensor assembly further includes a pair of convex lenses to focus an object image of tissue on the image sensor.

7. The colposcope according to any preceding claim, further comprising cables interconnecting the camera assembly and the handle assembly to transfer power and video data therebetween, the cables extending through a third lumen of the plurality of lumens.

8. The colposcope according to any preceding claim, wherein the camera assembly includes a hemispherical dome-shaped acrylic housing; and/or wherein the handle assembly further includes a motor operatively coupled with the articulation dial such that actuation of the motor through rotation of the articulation dial causes axial displacement of at least one of the articulation cables.

9. The colposcope according to any preceding claim, wherein the elongate member has a tubular structure.

10. The colposcope according to any preceding claim, wherein the elongate member is formed of silicone; and/or wherein the articulation dial is rotatable about a longitudinal axis defined by the elongate member.

11. The colposcope according to any preceding claim, wherein the handle assembly further includes a battery pack detachably received in the grip, the battery pack configured to supply power to the camera assembly.

12. The colposcope according to any preceding claim, wherein the image sensor of the camera assembly is a charge-coupled device.

13. A kit for performing colposcopy comprising:
a colposcope comprising:
a handle assembly including:
a grip;
a trigger movable towards and away from the grip; and
an articulation dial;
an elongate member extending distally from the handle assembly, the elongate member including lumens; and
a camera assembly including a neck portion coupled to the elongate member, and a body portion operatively coupled with the articulation dial, the body portion including an image sensor assembly having an image sensor and light emitters, the image sensor assembly operatively coupled with the trigger such that when the trigger is actuated the image sensor captures images of a target tissue; and
an electronic device connectable to the colposcope, wherein the electronic device is configured to display the target tissue.

14. The kit according to claim 13, wherein the electronic device is wirelessly connectable to the colposcope.

15. The kit according to claim 13 or claim 14, wherein the colposcope further includes a memory to store images of the target tissue captured by the image sensor; and/or wherein the camera assembly is movable between a first position, in which, the camera assembly is aligned with the longitudinal axis of the elongate member, and a second position in which at least a portion of the camera assembly is offset from the longitudinal axis of the elongate member.
